# EUROPEAN PATENT APPLICATION

(11) **EP 1 517 253 A2**
(43) Date of publication of application: **23.03.2005**
(21) Application number: 04020889.4
(22) Date of filing: 02.09.2004
(51) Int. Cl.: G06F 17/50

(54) **Method for design support of functional nucleic acids**

(30) Priority: 19.09.2003 JP 2003328264
(71) Applicant: Hitachi Software Engineering Co., Ltd., Yokohama-shi, Kanagawa 230-0045 (JP)
(72) Inventor: Kasai, Yasuhiro, Shinagawa-ku Tokyo 140-0002 (JP); Sugimoto, Takashi, Shinagawa-ku Tokyo 140-0002 (JP); Muto, Isamu, Shinagawa-ku Tokyo 140-0002 (JP); Iwao, Kanako, Shinagawa-ku Tokyo 140-0002 (JP); Kondo, Aiko, Shinagawa-ku Tokyo 140-0002 (JP)
(74) Representative: Liesegang, Eva

(57) **Abstract**

A visual interface that allows a user to designate intuitively a two-dimensional sequence suitable for a target sequence for a functional nucleic acid is provided. Secondary structure data of a target mRNA is displayed on a screen, and various information such as characteristic sequences to be effectively influenced by the functional nucleic acid, binding sites for protein motifs, and UTRs are displayed on the secondary structure. A target site of the functional nucleic acid is designated by the user with a pointing device, and the intuitive determination of a most suitable sequence is supported by repeating the design of the functional nucleic acid.

## Description

### FIELD OF THE INVENTION

The present invention relates to a method for design support of nucleic acids that function *in vivo.*

### BACKGROUND OF THE INVENTION

Nucleic acids are capable of forming their own conformation. Particularly, RNA with its high capability exists in nature as a nucleic acid that combines information and function, and functions *in vivo.* Since RNA was given another look as a functioning molecule, researches on the structure and the function of RNA have been actively conducted. Nucleic acids are functional molecules with simple structures that are made of only four kinds of bases, which is different from proteins composed of many kinds of amino acids. Hence, attention is being paid to nucleic acids from a standpoint of basic research including prediction of their secondary structures to be formed and elucidation of mechanism of enzyme activity based on their sequence information as well as that of applied research such as inhibition of gene expression with the use of nucleic acids, particularly RNA.

For a method to inhibit gene expression with RNA, ribozymes are well known. The typical ribozymes currently being used include hammerhead ribozymes and hairpin ribozymes. More recently, antisense method and RNAi method in which these RNAs function *in vivo* by making use of various intracellular mechanisms to suppress gene expression even if they themselves do not have any enzymatic activities to inhibit gene are rapidly becoming widespread. In addition, the method of inhibition of gene expression not only by RNA but also by DNA is being explored beginning with nucleic acid DNazyme that functions *in vivo.*

In accordance with the progress in biology, functional analysis of gene has come to require speed and accuracy more than ever. Analysis with the use of ribozyme, antisense method, and siRNA allows specifically inhibiting gene expression with great ease compared with conventional gene knockout technology, and it is a technology that currently attracts attention.

[Non-patent document 1] GENE SILENCING IN MAMMALS BY SMALL INTERFERING RNAS, Michael T. McManus and Phillip A. Sharp, Nat Rev Genet. 2002 Oct;3(10):737-47.

[Non-patent document 2] Ribozymes: A Modern Tool in Medicine, Asad U. Khan and Sunil K. Lal, J. Biomed. Sci., 2003 10:457-467

[Non-patent document 3] Thermodynamic criteria for high hit rate antisense oligonucleotide design., Matveeva OV, Freier SM., et al., Nucleic Acids Res. 2003 Sep 1;31(17):4989-94.

### SUMMARY OF THE INVENTION

Although the inhibition of gene expression with a nucleic acid that functions *in vivo* such as ribozyme is simple as an experimental technique, it is necessary to select a target sequence on which the nucleic acid that functions *in vivo* acts most efficiently from a target nucleic acid sequence. For example, the sequence on which ribozymes act efficiently in cells contains NUX sequence at the cleavage site, and is believed to reside in the 5' UTR region of the target sequence as well as in a region that does not assume a stem structure in its predicted secondary structure. The stem structure that represents one of the secondary structures of nucleic acids is considered to be a significant factor to inhibit binding of a nucleic acid that functions *in vivo* to its target. It is also important that the selected target site does not have a homology to mRNAs other than the target. Since substantial EST database is available for each species of organisms in recent years, it has become rather easy to search for a region having no homology to any sequences other than the target site.

Although many methods for designing nucleic acids that function in vivo are proposed at present based on the above information, a definitive method of designing the sequence has not yet been proposed. Therefore, the design of a nucleic acid that functions *in vivo* essentially requires designing of a plurality of sequences as well as repeating the design while confirming the effects of the designed sequences.

The object of the present invention is to provide a support method for determination work of a target sequence by a user so that the target sequence might be determined visually, intuitively, and efficiently by the user in the design of a nucleic acid that functions *in vivo* such as ribozyme.

To solve the above problems, the present invention supports the design of a nucleic acid that functions *in vivo* (hereinafter, referred to as functional nucleic acid) by steps of: focusing attention on the secondary structure of the target nucleic acid that gives an important effect on the activity of the functional nucleic acid; displaying various annotation information that the target sequence has on the secondary structure and restricting information of the functional nucleic acid to be designed for the target sequence; and then selecting visually and intuitively a sequence predicted to be effective from the nucleic acid sequence shown as the secondary structure in a way that the user designates with a pointing device.

Currently, there are a number of software applications that give rise to a design sequence by inputting conditions such as those mentioned above. However, information provided to users is only the nucleic acid sequence after completing the design in many cases. Therefore, it is thought necessary that a predicted secondary structure of the target and its associated information, both of which are useful for promoting the design by repetition, as well as the designed functional nucleic acid are displayed visually and intuitively at the same time.

In consideration of repeated design by a user, the present invention allows to design a functional nucleic acid by steps of: making the user strongly realize what kind of structure the target nucleic acid assumes *in vitro* or *in vivo*; displaying various information possessed by the target sequence on the secondary sequence structure; designating a candidate sequence with a pointing device; predicting an effect when the candidate sequence is selected for a target sequence; and carrying out the design by trial and error. Moreover, the present invention allows supporting judgment based on the user's experience by displaying also an output from existing software for predicting nucleic acid sequences on the secondary structure.

In other words, the method for design support of functional nucleic acid acting on a target nucleic acid according to the present invention includes steps of: displaying the secondary structure data of the target nucleic acid; discernibly displaying annotation information that represents characteristics of partial sequences constituting the target nucleic acid on the secondary structure data of the target nucleic acid by correlating with the corresponding partial sequences; and accepting the designation of a partial sequence on the displayed secondary structure data of the target nucleic acid as the target sequence on which the functional nucleic acid acts.

When the designated partial sequence does not meet the conditions for the target sequence of the functional nucleic acid, it is better to notify the above information in a manner such as highlight. Furthermore, it is preferred that a partial sequence meeting the conditions preset for the target sequence of the functional nucleic acid in the sequence of the target nucleic acid is discernibly displayed on the displayed secondary structure data of the target nucleic acid. The annotation information includes regions highly homologous to other ESTs, UTR regions, SNPs, etc. These pieces of annotation information may be varied depending on the kind of the functional nucleic acid. The functional nucleic acids aimed at in the present invention include, for example, ribozyme, siRNA, antisense RNA, and DNazyme.

The melting temperature and the GC content of the designated partial sequence may be displayed. The designation of the partial sequence on the secondary structure data of the target nucleic acid may be carried out by defining the length of functional nucleic acid in advance and indicating a starting point for the partial sequence. An arbitrary sequence that the user designated may be highlighted on the secondary structure data of the target nucleic acid.

The method of the present invention can be realized by a computer program.

According to the present invention, not only sequence information that represents a primary structure but also a secondary structure of the sequence information is focused attention on, and whether the target region for the nucleic acid to be designed is a region in a large open loop or an end region in a long stem structure can be distinguished. Furthermore, when the design region is practically determined, it is possible to design with the image of a state closer to reality by enabling the user to designate a sequence on the two-dimensional structure rather than the one-dimensional structure.

By employing such display method and sequence designation method, it is possible for the user to design more suitable sequences without being restrained by only linear images of nucleic acid sequences as before.

### BRIEF DESCRIPTION OF THE DRAWINGS

Fig. 1 is an explanatory drawing of a display example;
Fig. 2 represents a system configuration;
Fig. 3 is a process flow diagram;
Fig. 4 represents a structure of sequence information;
Fig. 5 represents a sequence structure to store a secondary structure of the sequence;
Fig. 6 represents a structure of annotation information;
Fig. 7 is a conceptual drawing of an interface of sequence design;
Fig. 8 is a display example of the annotation information;
Fig. 9 represents a structure of restricting information on a nucleic acid to be designed;
Fig. 10 is an example of designating a nucleic acid sequence; and
Fig. 11 represents a structure of information on a candidate sequence.

### DETAILED DESCRIPTION OF THE PREFERRED EMBODIMENTS

Embodiments of the present invention will hereinafter be explained with reference to the accompanying drawings.

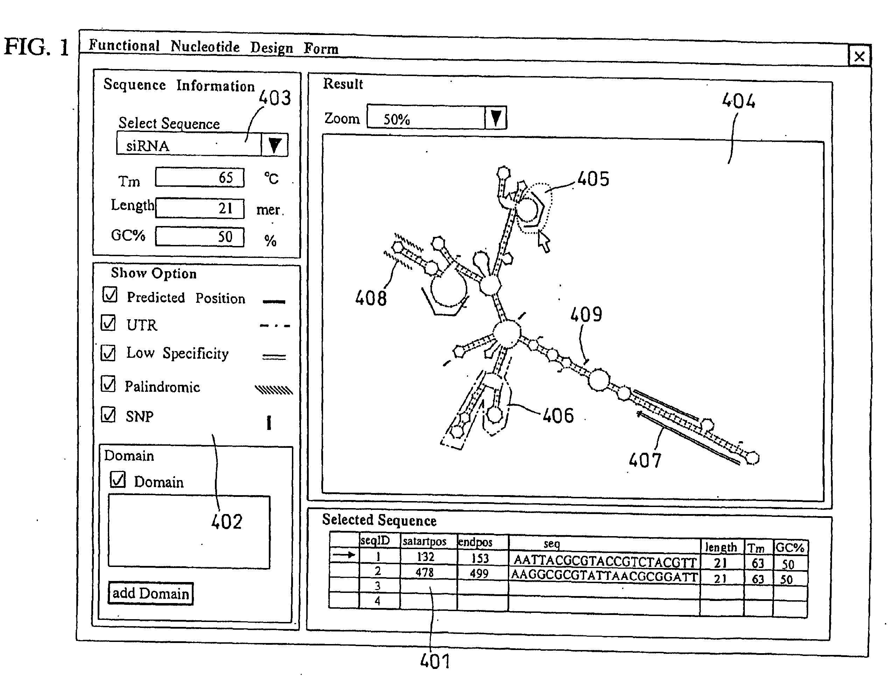

Fig. 1 represents an example of display according to the present invention. This display example is the one when siRNA is designed. Data of a secondary structure of a target gene sequence is displayed on a display region for secondary structure 404. On the display, a predicted region for the sequence 405 (Predicted Position), a UTR region 406 (UTR), a high homology region to other ESTs 407 (Low Specificity), a region with palindromic structure 408 (Palindromic), and a SNP 409 (snp) are displayed. Legends for display options 402 allows these displays using prediction software that stores sequence information necessary for ribozyme design.

It is possible to confirm visually in a comprehensive manner whether or not the candidate sequence in the target that has been predicted with the sequence prediction software is really acceptable by displaying it with information about peripheral regions on this screen. In this manner, the choice of whether to use the designed sequence or to make a modification to it, for example, to make the recognition sequence a little longer can easily be made.

In designing a functional nucleic acid that targets, for example, a motif region common to a certain group of genes, those parts of sequences with the common motif are cataloged as sequences and displayed on the screen. In this way, it is possible to visually design a region that seems likely to be particularly effective among the common motif sequences.

Furthermore, even when the design results in a palindromic sequence or a peculiar sequence such that stems of ribozymes bind to each other, easy mistakes made by users are prevented by displaying the designed region in a warning color. In addition, the design by users may be supported by calculating Tm and GC content of the region being selected and displaying them dynamically.

In Fig. 1, the display is designed so as to be switched between display/non-display for each case described above with the display option 402. Besides the display information mentioned above, it is possible for users to add sequences that exist in ESTs and bind to specific proteins and additional specific sequences that attract attention, and to display in the display region for secondary structure 404. In the embodiment shown in Fig. 1, the sequences desired to be displayed can be added by pressing a button "add Domain" present in the display option 402 and invoking an interface to add sequences.

First, a user inputs information about a sequence desired to be designed into a design sequence option 403. In the embodiment shown in Fig. 1, information necessary to design siRNA and hammerhead ribozyme that are representative sequences is cataloged in advance, and the kind of the sequence desired to be designed is selected in a combo box located in the design sequence option. The design sequence option 403 allows a melting temperature (Tm), a sequence length desired to be designed (length), and a GC content (GC%) to be designated. When more detailed parameters other than the above or new design sequence information is desired to be additionally set, a button "Option" located in the sequence design option 403 can be used. Besides the above information, the use of this button invokes an interface across which restricting information about the design sequence that must be satisfied by the sequence desired to be designed and the like can be input.

Next, the user selects an arbitrary region on secondary structure data of the sequence displayed on the display region for secondary structure 404 with a mouse, and carries out a design. The user can strongly realize the secondary structure of the target sequence by directly pointing at a region on the structure information with the mouse. Moreover, which part of the target sequence is targeted for the designed functional nucleic acid can be instantly grasped. In Fig. 1, the designed sequence information is displayed on designed sequence data 401. Since information that should be satisfied by the sequence desired to be designed has been registered, the user can examine whether the user-designed sequence differs from the information already registered immediately after selecting a region on the display region for secondary structure 404. When the information of the user-designed sequence significantly differs from the information already registered or misfits the condition that needs to be satisfied, a notice is given to the user by highlighting of its sequence on the secondary structure of the sequence or the designed sequence data 401. In the present embodiment, that notice is given when the Tm, GC content (GC%), and length differ by ± 5 degrees C, ± 5%, and one or more nucleic acids, respectively, from their pre-registered values. Alternatively, this highlighted notice may employ a mode that the notice is given to the user when the conditions are met and is not given when the conditions are not met.

According to the present invention, it is possible to design a region such as a boundary region between a stem region and a loop region of a nucleic acid while having an image as shown in Fig. 1. Since the present design method is not perfect, an interface that makes it easy to design repeatedly in consideration of the structure is useful in the present design method that repeats trial and error.

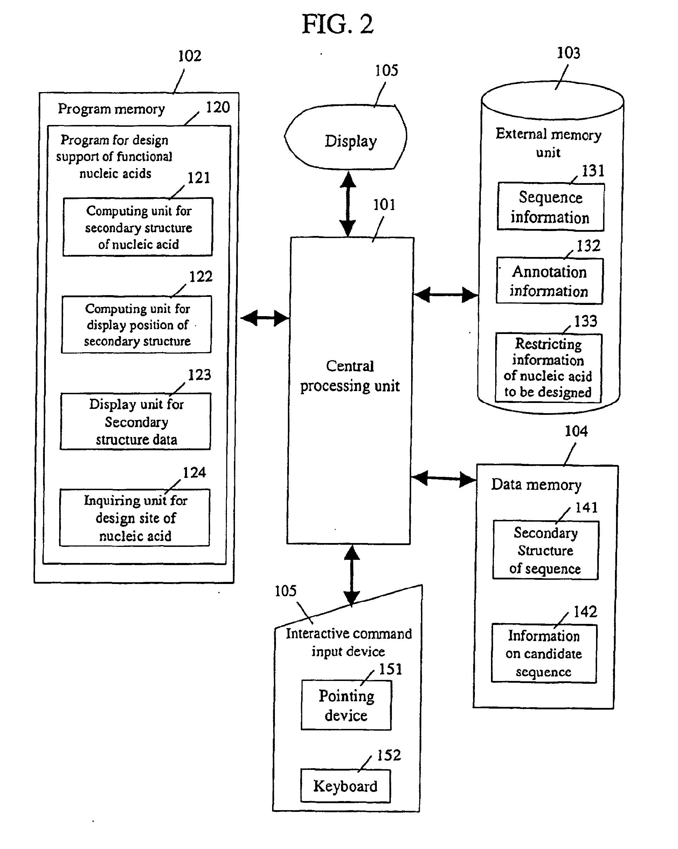

Fig. 2 depicts a system configuration that is used to realize an interface for nucleic acid design according to the present invention. This system is composed of a central processing unit 101 that carries out processing of secondary structures of nucleic acids and of how to arrange the secondary structures on the screen, a program memory 102 that stores a program for allowing the central processing unit 101 to execute the processing, an external storage unit 103 such as a hard disk, a data memory 104 that temporarily stores data, an input device 105 to input values into the system and command selection operations, and a display 106 that realizes an interaction with a user.

A program for design support of functional nucleic acid 120 of the present invention is stored in the program memory 102. The program for design support of functional nucleic acid 120 is composed of programs such as a computing unit for secondary structure of nucleic acid 121 to compute a secondary structure of a target nucleic acid, a computing unit for display position of secondary structure 122 to appropriately arrange the computed secondary structure on the screen, a display unit for secondary structure data of nucleic acid 123 to display the computed secondary structure in a practically understandable way to users, and an inquiring unit for design site of nucleic acid 124 that inquires the users about a design site of the nucleic acid. These programs may be provided by being stored in a recording medium such as a floppy (trademark) disk, CD-ROM, DVD-ROM, or MO. In addition, these programs may also be provided through the network.

The external storage unit 103 stores sequence information 131 of a target nucleic acid or nucleic acids that are not targeted but are likely to interact with the designed sequence, annotation information 132 associated with the sequence that represents additional information to display on the secondary structure, and restricting information of nucleic acid to be designed. The data memory 104 stores secondary structure of sequence 141 in which computed secondary structures of nucleic acids are stored and information on candidate sequence 142 in which layout information of the secondary structures on the screen is stored. The input device 105 is composed of a pointing device 151, a keyboard 152, and the like. Although the program memory 102 and the data memory 104 are separately depicted in Fig. 2, these may be physically one memory as a matter of course.

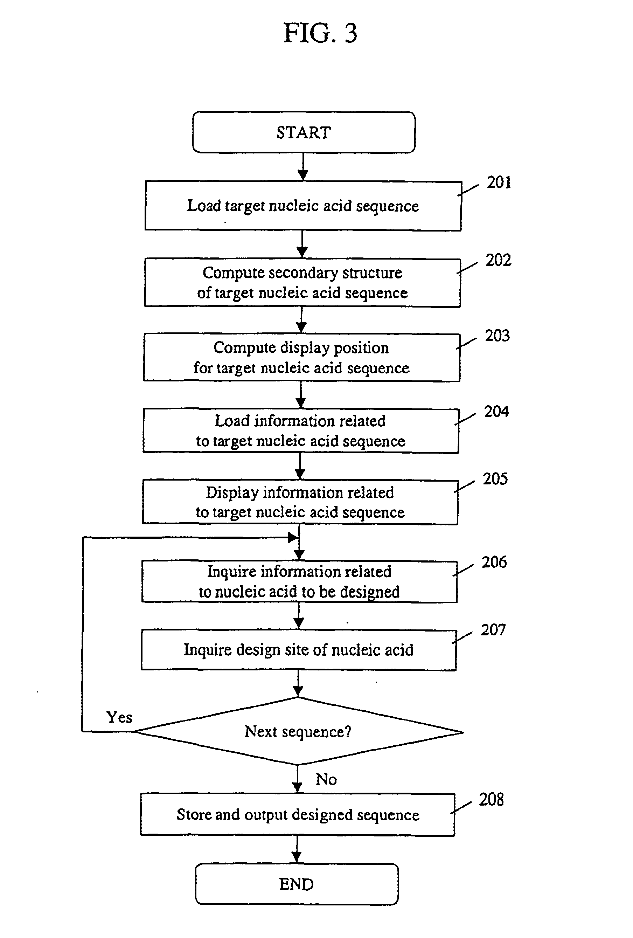

Fig. 3 is a processing flow for users to carry out design of nucleic acid sequence with the use of the pointing device 151 by displaying various annotation information on the secondary sequence information of a nucleic acid based on the sequence information 131, the annotation information 132, and restricting information of nucleic acid to be designed 133.

When the processing is started, the sequence of a gene whose expression users want to control is first loaded from the sequence information 131 at a step 201. In many cases, the target sequence is one that is present *in vivo* as an mRNA in various living organisms.

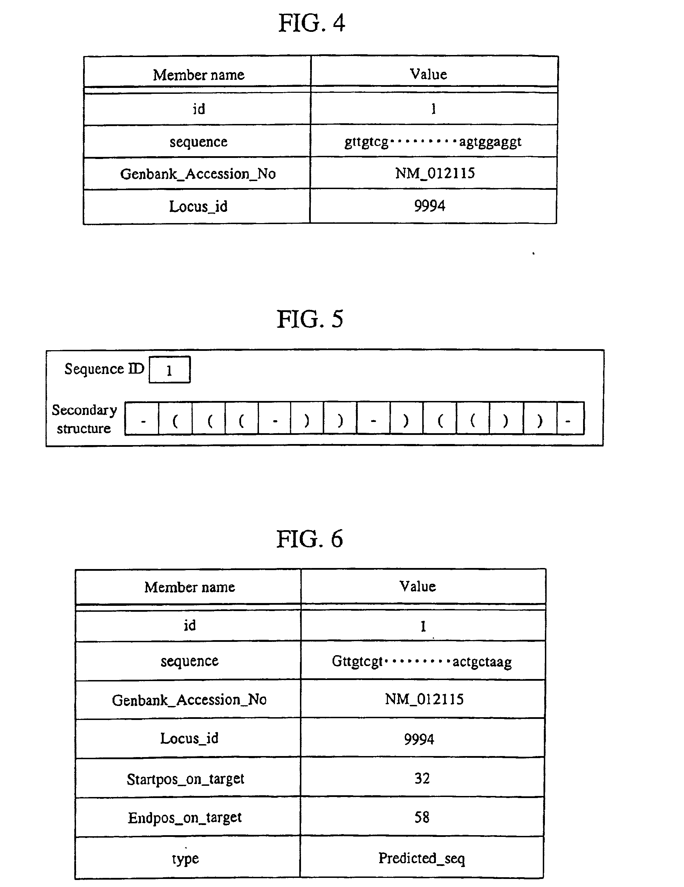

A specific structure of the sequence information 131 is shown in Fig. 4. The sequence information is stored as a structure in the present invention. That is, a gene sequence (sequence) with a sequence ID (id), its related representative Genbank Accession No (Genbank_Accession_No) and Locus Link ID (Locus_id) that are keys to the external database, are stored in one structure.

Next, at a step 202, the secondary structure of sequence 141 is computed for the loaded sequence data by the central processing unit 101 using the computing program for secondary structure of nucleic acid 121. A number of investigations have been conducted so far as to the computation of secondary structures of nucleic acids, and any program of M. Zucker's M-FOLD, Vienna's RNA package, or the like may be used for the computation of the secondary structure. The computation of secondary structures of nucleic acids needs temperature, salt concentration, and the like as parameters for their structure computation. At this step, users are asked about these parameters on the display 106 as required, and these are set up using the pointing device 151 and the keyboard 152. At this time, various parameters may also be asked by the central processing unit. At a subsequent step 203, a computation of display position for the target nucleic acid sequence on the screen is performed.

A specific structure of the secondary structure of sequence 141 is shown in Fig. 5. How to express the secondary structure of a sequence in a more efficient way is being approached as a field of study. In the present invention, the secondary structure is expressed by three kinds of punctuation symbols, i.e. "(", "-", and ")", in the primary sequence. A smaller number of subscript for sequence corresponds to 5' side of the sequence. The "-" represents a base not involved in a base pair formation, and the "(" represents a base that forms a base pair with ")". Although such a data structure is employed for the sake of convenience in the present invention, any method may be used to express the secondary sequence structure.

At a next step 204, the annotation information 132 is loaded. Hereby, the annotation information consists of data representing part of nucleic acid sequence and data of its associated information. Conceivable information includes information about UTRs known for the target nucleic acid, SNPs, sequences highly homologous to other various nucleic acid sequences *in vivo* including mRNA, and sequences seen in certain specific kinds of gene sequences in common, and the like. In addition, sequences of functional nucleic acids predicted beforehand with the use of other software to predict a functional nucleic acid sequence may be used for the annotation information.

The specific structure of the annotation information 132 is shown in Fig. 6. In the present invention, the annotation information is retained as one structure. That is, the annotation information is stored in one structure including, as essential items, id (id) that allows a unique identification of the annotation information, sequence information (sequence), a starting position on the target sequence (startpos_on_target), an end position on the target sequence (endpos_on_target), and a type representing the kind of annotation (type), and as additional items, Genbank Accession No (Genbank_Accession_No) and Locus Link ID (Locus_id) that are keys to representative biological database accessible from any computer. Further information may also be added.

At a subsequent step 205, information related to the target nucleic acid sequence is displayed. At the step 205, all information specified so far is displayed on the secondary structure of the target nucleic acid that has been computed at the step 203.

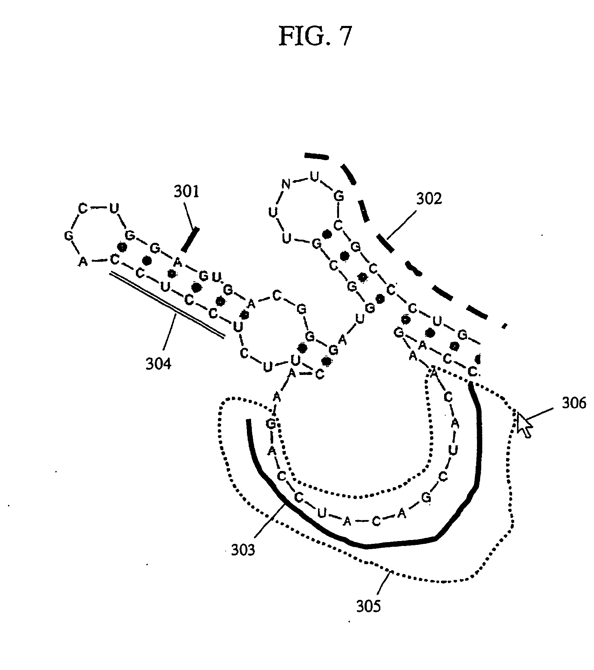

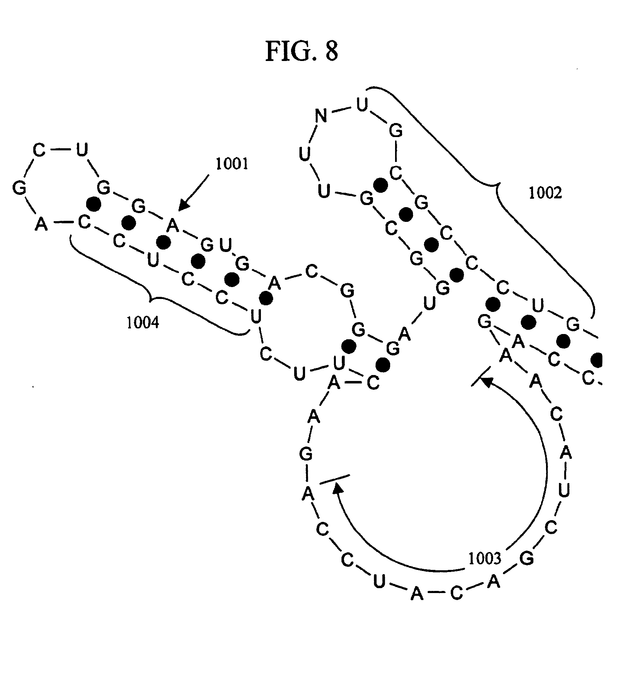

Fig. 7 is a display example of the secondary sequence data of the target nucleic acid sequence and its associated annotation information. The annotation information is expressed by drawing a line beside each corresponding base on the secondary structure data of the target nucleic acid sequence. A SNP 301 is shown with a line perpendicular to a base, a UTR 302 with a bold dotted line, a predicted target site 303 with a bold solid line, and a region highly homologous to other mRNA in living organisms 304 with a double line, respectively. In the display example, annotation information is each shown on the secondary structure of sequence data so as to be recognized at a glance by changing the kinds of lines for each kind of annotation information. The display method of various annotation sequences may employ not only the method of changing the kinds of lines but also a method of changing line colors for each annotation and a method of discerning with the use of different letter colors for each annotation as shown in Fig. 8. The kinds of the annotation are discerned by letter colors in Fig. 8. SNP 1001 is displayed in lime green, UTR 1002 in light blue, the predicted target site sequence 1003 in blue, and the region highly homologous to other mRNA in living organisms 1004 in red, thereby allowing discerning the annotation information.

At a next step 206, information on a functional nucleic acid to be designed is specified. As to the restricting information on the nucleic acid to be designed 133, its relevant information to be specified includes general information such as the length of the nucleic acid to be designed, and the target Tm value and GC content. In the case of designing a ribozyme, information on the presence of NUX at a sequence cleavage site is specified. In the case of information on siRNA, beginning of its sequencing from 5'-AA is specified. When users conduct the design repeatedly, data at this step change most frequently. Therefore, an interface for directly inputting values as well as the input from the external storage unit 103 may be provided.

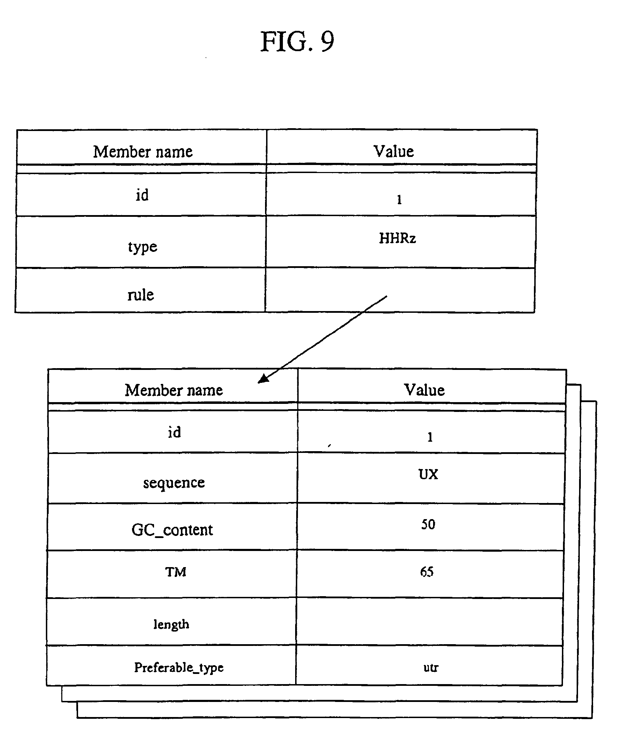

A specific structure of the restricting information on nucleic acid to be designed 133 is shown in Fig. 9. In the present invention, conditions of designing sequences are retained as a structure. That is, the structure is composed of id (id) that allows a unique identification of the restricting information, the type of design sequence (type), and the practical restricting information (rule). The type of design sequence is specified with a letter string that is readily understood as to what is designed. For example, when the design sequence is a hammerhead ribozyme, the letter string is HHRz, and when it is a siRNA, the letter string is siRNA. Since the restricting information is conceivable not to be a piece but multiple pieces, a front pointer to the structure arrangement storing detailed restricting information is reserved in the present invention. The detailed restricting information stores detailed rule of the restricting information. That is, the detailed rule is composed of id (id) that allows a unique identification of the rule, essential sequence information (sequence), an ideal GC content (GC_content), TM (TM), length of sequence (length), and preferable annotation to be targeted (Preferable_type). The essential sequence information indicates sequence information essentially required for the type of design sequence (type). UX in Fig. 9 indicates that 5'-UX-3' must be present in the design sequence. N representing any one of bases is unnecessary to write in. However, when it is shown that 19 consecutive pieces of N representing any base are interposed between two consecutive A on the 5' side and two consecutive T at the end as in the case of siRNA design, N is explicitly written in like AA(N19)TT. Although the items as above are exemplified as the detailed restricting information in the present invention, it is allowable to add any items to that information.

At a step 207, a design site of nucleic acid is asked to a user. An example of designation at the time of designating a nucleic acid sequence is shown in Fig. 7. Based on the sequence displayed on the screen and the annotation information, the user designates a design site in the nucleic acid sequence using the pointing device 151 such as a mouse 306. The method of designating the design site is the one in which a region 305 is designated while keeping a button of the pointing device 151 pressed down. In a state that the button of the pointing device 151 is kept pressed down, the region is designated so as to follow the track of the pointing device 151 from the position where the button was initially pressed down. The bases included in the region represent the design sequence. The method of the designation with the pointing device may differ from the above method. For example, a method of designation by clicking the initial base and the last base is conceivable. Furthermore, the design sequence may be designated by specifying the length of the design sequence beforehand in the restricting information on each functional nucleic acid and by clicking the base at the initial position.

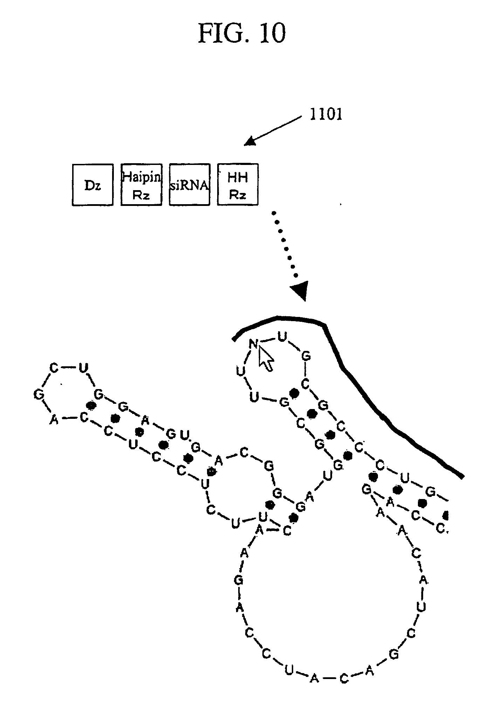

When functional nucleic acids are designed, the procedure of the step 207 may also be carried out by the following method: Icons 1101 that indicate setting of multiple kinds of functional nucleic acids are prepared as shown in Fig. 10, and the restricting information 133 is selected by dragging the icon, followed by dropping it at the base site of design initiation in the sequence. At this time, since the restricting information on various functional nucleic acids is specified in advance at the step 206, the user can design any selected functional nucleic acid by the operation to drop the icon 1101, showing which functional nucleic acid is designed, at the base site of design initiation. Fig. 10 depicts a state that the icon 1101 representing the functional nucleic acid shown by HHRz (hammerhead ribozyme) is dragged with a mouse and is going to be dropped on a sequence of the secondary structure. The dotted line in Fig. 10 indicates a movement of the mouse cursor for convenience sake. Since the length of HHRz desired to be designed has already been input at the step 206, a bold line is drawn on the secondary structure data by the length of HHRz desired to be designed from the position of the mouse cursor in Fig. 10.

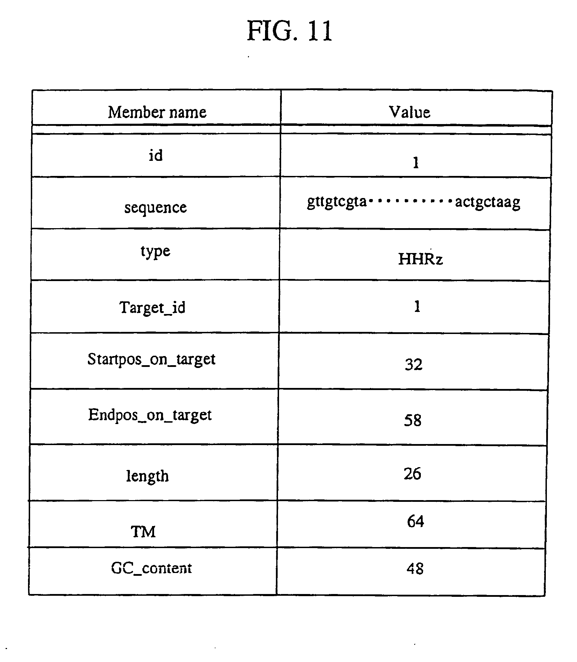

Fig. 11 depicts a specific structure of information about the candidate sequence actually designed by the user. In the present invention, the information about the candidate sequence designed by the user is retained as one structure. That is, the structure is composed of id (id) that allows a unique identification of the sequence, a designed sequence (sequence), a type of the design sequence (type), id to specify a target sequence (Target_id), a position on the target sequence (Startpos_on_target, Endpos_on_target), length of the sequence (length), TM (TM), and GC content (GC_content).

When a region is designated, the sequence of the designated region is highlighted on the screen to notify the user if the information differs from that designated for TM, length of sequence, and the like at the step 206. In the example shown in Fig. 1, the letters on the designed sequence data 401 are highlighted by being made bold. This notification may be contained in a pop-up window or the like to give a warning immediately after the user has selected the region. Owing to this display, the user can judge whether to design the sequence at a different position or design it at the same position by elongating a few more number of bases for the sequence based on the displayed information.

At the time of designating a region, the designation of a sequence may be made not to select bases longer than the indicated length of sequence when designing a sequence with a definite length of sequence such as siRNA.

The user can finally determine the design sequence after much trial and error on the basis of the annotation information about the predicted site and the sequence. When multiple sequences are designed for the same target, the sequence design can be repeated by going back to the step 206. When the target sequence is changed, the design can be repeated by going back to the step 201. The sequences designed in this way are stored in the form of the structure shown in Fig. 11 at a step 208 and are output.

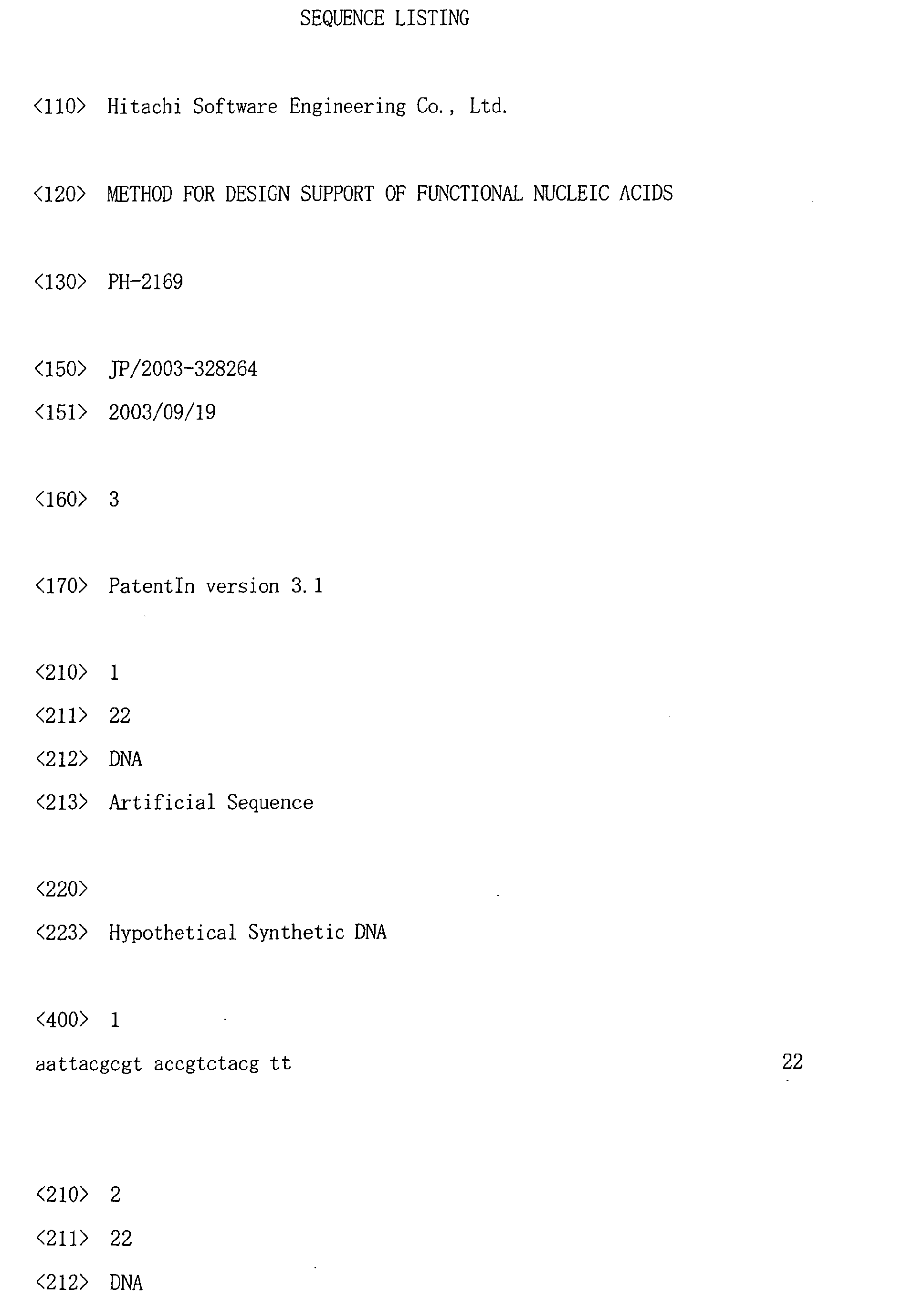

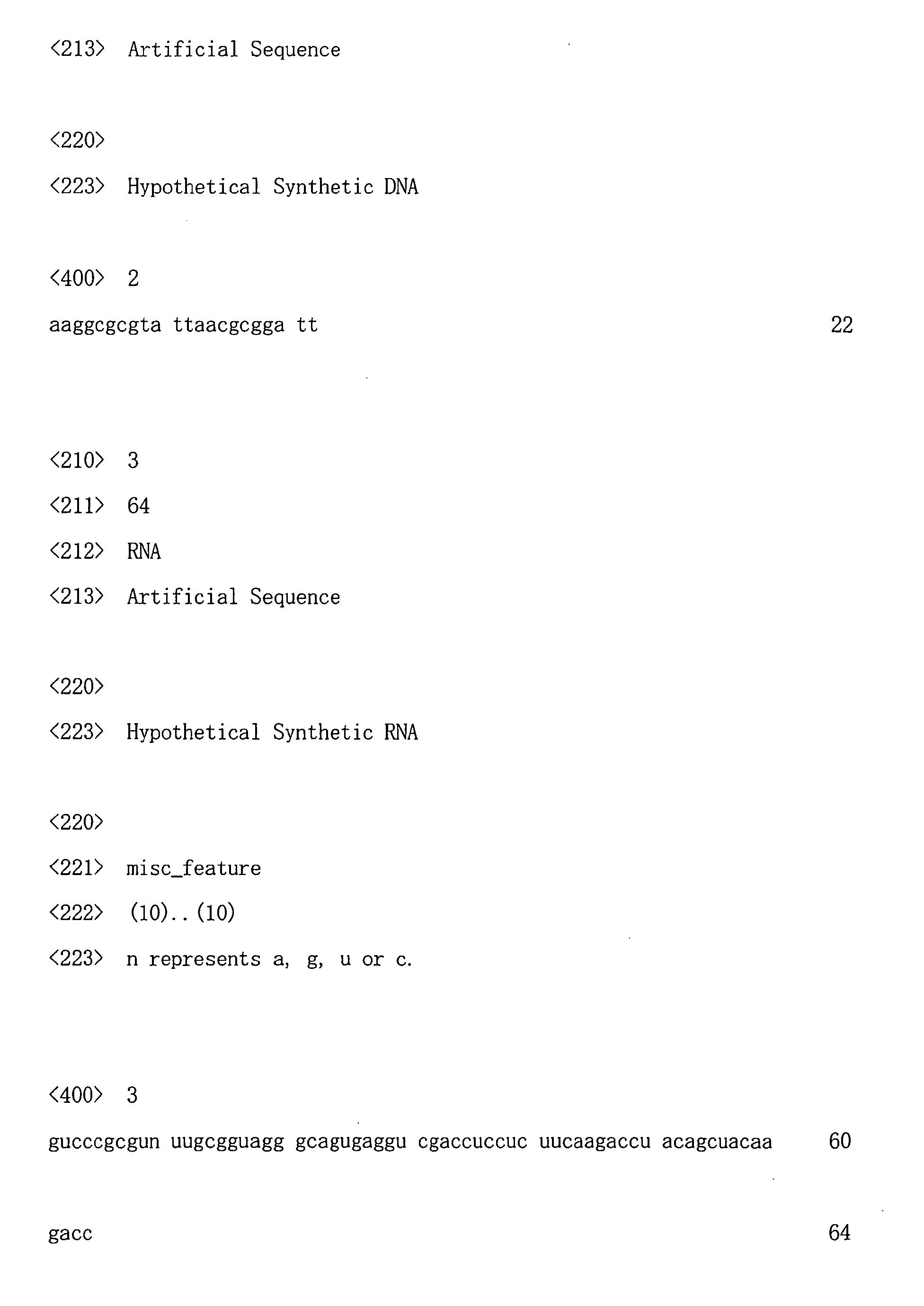

## Claims

1. A method for design support of functional nucleic acids to act on a target nucleic acid, comprising steps of:
displaying a secondary structure data of the target nucleic acid;
discernibly displaying annotation information that represents characteristics of partial sequences constituting the target nucleic acid on the secondary structure data of the target nucleic acid by correlating with the corresponding partial sequences; and
accepting the designation of a partial sequence on the displayed secondary structure data of the target nucleic acid as a target sequence on which the functional nucleic acid acts.

2. The method for design support of functional nucleic acids to act on a target nucleic acid according to claim 1, wherein when the designated partial sequence does not meet the conditions for the target sequence of the functional nucleic acid, the information is notified.

3. The method for design support of functional nucleic acids to act on a target nucleic acid according to claim 1, wherein the partial sequence meeting the conditions preset for the target sequence of the functional nucleic acid in the sequence of the target nucleic acid is discernibly displayed on the displayed secondary structure data of the target nucleic acid.

4. The method for design support of functional nucleic acids to act on a target nucleic acid according to claim 1, wherein the annotation information includes regions highly homologous to other ESTs, UTR regions, and SNPs.

5. The method for design support of functional nucleic acids to act on a target nucleic acid according to claim 1, wherein the annotation information is varied depending on the kind of the functional nucleic acid.

6. The method for design support of functional nucleic acids to act on a target nucleic acid according to claim 1, wherein the functional nucleic acid is any one ofribozyme, siRNA, antisense RNA, and DNazyme.

7. The method for design support of functional nucleic acids to act on a target nucleic acid according to claim 1, wherein the melting temperature and the GC content of the designated partial sequence are displayed.

8. The method for design support of functional nucleic acids to act on a target nucleic acid according to claim 1, wherein information on the length of the functional nucleic acid is provided in advance and the designation of the partial sequence on the secondary structure data of the target nucleic acid is carried out by indicating a starting point for the partial sequence.

9. The method for design support of functional nucleic acids to act on a target nucleic acid according to claim 1, wherein an arbitrary sequence designated by a user is highlighted on the secondary structure data of the target nucleic acid.

10. A computer program loadable into the memory of a computer, comprising software code for performing the method of one of the preceding claims when run on the computer, wherein
the secondary structure data of the target nucleic acid is stored in a memory of the computer and is displayed on a display connected to the computer; and said annotation information is stored in the memory of the computer.
